# EUROPEAN PATENT SPECIFICATION

(11) **EP 2 986 937 B1**
(45) Date of publication and mention of the grant of the patent: **01.03.2017**
(21) Application number: 14713104.9
(22) Date of filing: 26.03.2014
(51) Int. Cl.: G01B 21/30, A61B 5/00, G01B 5/28

(54) **METHOD FOR ASSESSING THE STATE OF HAIR**
VERFAHREN ZUR HAARZUSTANDSBESTIMMUNG
PROCÉDÉ POUR DÉTERMINER L'ÉTAT DE CHEVEUX

(30) Priority: 19.04.2013 EP 13164541
(43) Date of publication of application: 24.02.2016
(73) Proprietor: Unilever PLC, London, Greater London EC4Y 0DY (GB); Unilever N.V., 3013 AL Rotterdam (NL)
(72) Inventor: FESSI, Myriam, Wirra Merseyside CH63 3JW (GB); WIRE, Stephen, Lee, Wirra Merseyside CH63 3JW (GB)
(74) Representative: Chisem, Janet
(86) International application number: PCT/EP2014/056104
(87) International publication number: WO 2014/170108

(56) References cited:
- WO-A2-02/24071
- JP-A- 2004 195 722
- BERGMANN TIEST W M ET AL: "Analysis of haptic perception of materials by multidimensional scaling and physical measurements of roughness and compressibility", ACTA PSYCHOLOGICA, NORTH-HOLLAND, AMSTERDAM, NL, vol. 121, no. 1, 1 January 2006 (2006-01-01), pages 1-20, XP027908496, ISSN: 0001-6918 [retrieved on 2006-01-01]
- BHM Black Hair Media - Brenda Barrett: "How To: Tell If Your hair Is Damaged", , 13 April 2012 (2012-04-13), XP002698004, Retrieved from the Internet: URL:http://blackhairmedia.com/hair-care/ho w-to-tell-if-your-hair-is-damaged/ [retrieved on 2013-05-31]
- ZEMETRICS: "Surface measurement", , 6 February 2012 (2012-02-06), XP002698005, Retrieved from the Internet: URL:http://zemetrics.com/surface-roughness -measurement.shtml [retrieved on 2013-05-31]

## Description

The present invention relates to a method of assessing the state of a user's hair, using a self-assessment device.

The prior art discloses methods of accessing damage to hair.

BHM Black Hair Media - Brenda Barrett: "How To: Tell If Your hair Is Damaged" discloses a number of methods to check if your hair is damaged. These include picking up a strand of hair and running it through your fingers to check whether it feels rough or smooth, looking at your hair brush to see if hair is breaking off in the middle or on the ends, checking split ends, checking how much and how quickly the hair can absorb water and checking whether your hair snaps after stretching.

WO 02/24071 A2 discloses a method for measuring friction in a hair sample, comprising: (a) providing a friction member; (b) drawing it through the hair, generating a frictional noise signal; and (c) capturing the signal by a noise sensor. Also disclosed is a device suitable for use in said method, comprising comb means having a plurality of tines and a noise sensor arranged to capture frictional noise generated by passage of comb means through the hair. Finally disclosed is a system for assessing the level of damage in a hair sample, comprising: (a) defining hair categories; (b) associating with each category a standard trace representative of the frictional noise signal generated when a standard sample in that category is subjected to said method; (c) assigning the sample to one of the categories; (d) carrying out said method on the sample; (e) visually displaying the frictional noise signal generated as a trace; (f) and comparing the sample's trace with the standard trace associated with the category.

Bergmann Tiest W M et al; "Analysis of haptic perception of materials by multidimensional scaling and physical measurements of roughness and compressibility", Acta Physchologica, 121 (2006) 1-20, discloses a test where a series of textured stimuli were blind sorted by the use of touch, according to how similar they felt.

JP 2004 195722 A discloses a calibration method used in the production of a hard copy image of an object that has surface roughness. A plurality of roughness patches each having a different surface roughness is formed and a roughness correcting means utilises the reflection of light from the patches to calibrate and measure the roughness.

Zemetrics: XP-002698005, "Surface roughness measurement, surface metrology applications for optical profilers " describes optical surface measurement techniques for surfaces such as stents, dental implants and artificial bone. An example is a step height calibration standard, which is made of quartz and then chrome plated. Such items may have aspects of shape, roughness, surface finish and surface asperity.

Despite the prior art there remains a need for accurate yet simple means for self-assessing the state of hair fibres.

Accordingly, there is provided a method for assessing the state of hair according to claim 1.

The device allows for simple self-assessment of the state of the user's or another person's hair.

Preferably, the series comprises a series of patches of varying surface roughness. Preferably, the series comprises a series of patches of increasing surface roughness. This facilitates the user's ability to find the appropriate comparison with a hair sample.

Surface texture has elements of lay (the machining or forming pattern), surface roughness, and waviness. In addition, inherent material properties may contribute to surface porosity, inclusions, and residual elements. The parameters of texture are vertical amplitude variations, horizontal spacing variations, or some hybrid combination of these. Surface roughness is an expression of finely spaced vertical surface irregularities, as opposed to waviness, which is irregularities with spacing greater than surface roughness.

Surface roughness is provided by the presence of vertical surface irregularities or friction providing means.

Preferably the surface irregularities are in the form of raised or depressed features. Such features may be regularly or irregularly shaped and may be regularly or irregularly presented.

For example, the raised or depressed features may be circular, oval, square or rectangular. The raised or depressed features may be spaced regularly such that they are patterned or, preferably, they are spaced randomly.

Preferably, at least one of the patches comprises a plurality or raised features to generate a surface which is rough to the touch.

Preferably, the friction providing means is a patch which requires extra force to pass the finger over it. For example the surface might be tacky or abrasive to the touch. The patch may be provided by a painted or otherwise applied patch of abrasive or tacky material to the surface of the device.

Preferably at least one of the patches comprises no or substantially no raised features. This provides the user with a benchmark comparable to perfectly smooth hair.

Preferably, the series comprises from 2 to 16 patches, more preferably from 6 to 10.

Preferably, the patches are from 1 to 10 cm², preferably from 2 to 6 cm².

Preferably, the method further comprises making a product recommendation based on the score generated by the self-assessment. For example, should the assessor judge that the hair is damaged then the recommendation might be to recommend a composition suitable for damage repair.

Embodiments of the invention will now be described with reference to the following non-limiting drawings in which:
Figure 1 is a perspective view of a card, for use in the method of the invention, being used;
Figure 2 is a plan view of a similar device;

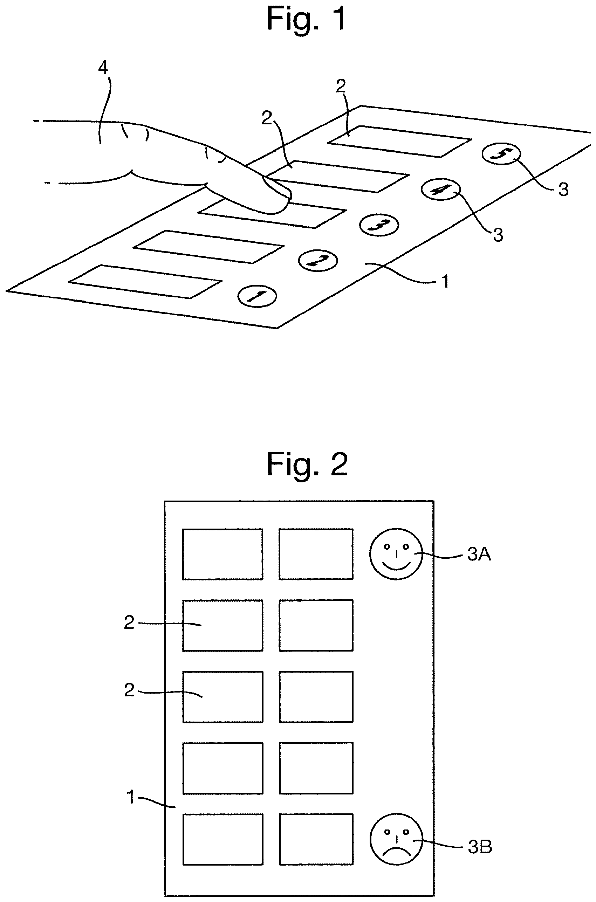

In detail, figure 1 shows a card (1) with a series of roughness patches (2) and indicators (3) which indicate to the assessor the roughness of each patch. The assessor's finger (4) is rubbed over the patches until the assessor concludes on the roughness patch with equates best with the roughness of the hair sample being assessed.

Figure 2 shows a similar card (1) in plan view. The indicators are caricatures indicating a positive (3A) or a negative (3B) result depending on roughness.

## Claims

1. A method for assessing the state of hair, using a self assessment device, said device comprising a series of rough patches of increasing roughness and which may be contacted by user and compared with the roughness of a hair sample in order to provide an indication to the user as to the relative state of the hair sample, by performing the steps of
- contacting a hair sample (step A), and
- contacting one or more rough patches on the device (step B) in that order or in reverse order,
- optionally repeating step A or step B before
- concluding as to which of the rough patches has the most similar roughness to the hair sample,
wherein the contacting is by the user's finger.

2. Method according to claim 1 wherein at least one of the patches of the device comprises vertical surface irregularities which is rough to the touch.

3. Method according to claim 1 or 2 wherein at least one of the patches of the device comprises no or substantially no surface texture.

4. Method according to any preceding claim wherein, the series of the device comprises from 2 to 16 patches, more preferably from 6 to 10.

5. Method according to any of claims 2-4 wherein the patches of the device are from 1 to 10 cm², preferably from 2 to 6 cm².

6. Method as claimed in any preceding claim, which further comprises making a product recommendation based on the score generated by the self-assessment.

## Patentansprüche

1. Verfahren zum Bewerten des Zustands von Haar unter Verwendung einer Selbsteinschätzungsvorrichtung, wobei die Vorrichtung eine Reihe von rauen Feldern mit zunehmender Rauigkeit umfasst, die durch den Benutzer berührt werden können und mit der Rauigkeit einer Haarprobe verglichen werden können, um einen Hinweis für den Benutzer über den relativen Zustand der Haarprobe bereitzustellen, indem die folgenden Schritte durchgeführt werden:
- Berühren einer Haarprobe (Schritt A) und
- Berühren eines rauen Feldes oder mehrerer rauer Felder auf der Vorrichtung (Schritt B)
in dieser Reihenfolge oder in der umgekehrten Reihenfolge,
- auf Wunsch Wiederholen von Schritt A oder Schritt B vor dem
- Schlussfolgern, welches der rauen Felder eine Rauigkeit aufweist, die der Haarprobe am nächsten kommt,
wobei die Berührung durch die Finger des Benutzers erfolgt.

2. Verfahren nach Anspruch 1, wobei wenigstens eines der Felder der Vorrichtung vertikale Oberflächenunregelmäßigkeiten umfasst, die bei der Berührung rau erscheinen.

3. Verfahren nach Anspruch 1 oder 2, wobei wenigstens eines der Felder der Vorrichtung keine oder im Wesentlichen keine Oberflächentextur umfasst.

4. Verfahren nach einem der vorhergehenden Ansprüche, wobei die Reihe der Vorrichtung 2 bis 16 Felder, stärker bevorzugt 6 bis 10 Felder umfasst.

5. Verfahren nach einem der Ansprüche 2-4, wobei die Felder der Vorrichtung eine Größe von 1 bis 10 cm², vorzugsweise von 2 bis 6 cm² aufweisen.

6. Verfahren nach einem der vorhergehenden Ansprüche, das ferner das Äußern einer Produktempfehlung auf Basis der Auswertung, die durch die Selbsteinschätzung erzeugt wird, umfasst.

## Revendications

1. Procédé d'évaluation de l'état de cheveux, utilisant un dispositif d'auto-évaluation, ledit dispositif comprenant une série de pastilles rugueuses de rugosité croissante et lequel peut être mis en contact par l'utilisateur et comparé avec la rugosité d'un échantillon de cheveu afin de fournir une indication à l'utilisateur comme l'état relatif de l'échantillon de cheveu,
en réalisant les étapes de
- mise en contact d'un échantillon de cheveu (étape A), et
- mise en contact d'une ou plusieurs pastilles rugueuses sur le dispositif (étape B),
dans cet ordre ou dans l'ordre inverse,
- répétition éventuelle de l'étape A ou de l'étape B avant de
- conclure laquelle des pastilles rugueuses présente la rugosité la plus similaire à celle de l'échantillon de cheveu,
dans lequel la mise en contact se fait par le doigt de l'utilisateur.

2. Procédé selon la revendication 1, dans lequel au moins une des pastilles du dispositif comprend des irrégularités de surface verticales qui sont rugueuses au toucher.

3. Procédé selon la revendication 1 ou 2, dans lequel au moins une des pastilles du dispositif ne comprend pas ou pratiquement pas de texture de surface.

4. Procédé selon l'une quelconque des revendications précédentes, dans lequel la série du dispositif comprend de 2 à 16 pastilles, encore mieux de 6 à 10.

5. Procédé selon l'une quelconque des revendications 2-4, dans lequel les pastilles du dispositif sont de 1 à 10 cm², de préférence de 2 à 6 cm².

6. Procédé selon l'une quelconque des revendications précédentes, lequel comprend de plus une recommandation de produit sur la base du score produit par l'auto-évaluation.
